# FASCICULE DE BREVET EUROPEEN

(11) **EP 1 819 824 B1**
(45) Date de publication et mention de la délivrance du brevet: **02.12.2009**
(21) Numéro de dépôt: 05850443.2
(22) Date de dépôt: 07.12.2005
(51) Int. Cl.: C12N 15/88, C07C 43/13, C07H 3/00, C07F 9/141, C07C 237/08

(54) **COMPOSES ANALOGUES DE LIPIDES MEMBRANAIRES D'ARCHAEBACTERIES ET COMPOSITIONS LIPOSOMIALES INTEGRANT DE TELS COMPOSES**
ZU LIPIDMEMBRANEN IN ARCHEABAKTERIEN ANALOGE VERBINDUNGEN UND LIPOSOMALE ZUSAMMENSETZUNGEN MIT DIESEN VERBINDUNGEN
COMPOUNDS ANALOGOUS TO LIPID MEMBRANES IN ARCHAEBACTERIA AND LIPOSOMAL COMPOSITIONS INCLUDING SAID COMPOUNDS

(30) Priorité: 07.12.2004 FR 0413028
(43) Date de publication de la demande: 22.08.2007
(73) Titulaire: ECOLE NATIONALE SUPERIEURE DE CHIMIE DE RENNES, 35700 Rennes Cedex (FR); Université de Bretagne Occidentale, 29269 Brest (FR)
(72) Inventeur: BENVEGNU, Thierry, F-35700 Rennes (FR); PLUSQUELLEC, Daniel, F-35230 Noyal Chatillon Sur Seiche (FR); RETHORE, Gildas, F-35600 Redon (FR); SACHET, Mickaëlle, F-72000 Le Mans (FR); FEREC, Claude, F-29470 Plougastel-daoulas (FR); MONTIER, Tristan, F-29200 Brest (FR); DELEPINE, Pascal, F-29820 Bohars (FR); LEHN, Pierre, F-29200 Brest (FR)
(74) Mandataire: Larcher, Dominique
(86) Numéro de dépôt international: PCT/EP2005/056555
(87) Numéro de publication internationale: WO 2006/061396

(56) Documents cités:
- WO-A-00/64858
- WO-A-97/22333
- DE ROSA M: "Archaeal lipids: Structural features and supramolecular organization" THIN SOLID FILMS, ELSEVIER-SEQUOIA S.A. LAUSANNE, CH, vol. 284-285, 15 septembre 1996 (1996-09-15), pages 13-17, XP004078130 ISSN: 0040-6090

## Description

La présente invention concerne le domaine pharmaceutique.

Plus précisément l'invention concerne de nouveaux composés synthétiques analogues de lipides membranaires d'archaebactéries.

L'invention concerne également de nouvelles compositions liposomiales mettant en oeuvre de tels composés ainsi que l'utilisation de telles compositions pour la vectorisation de molécules d'intérêts thérapeutiques et/ou la vectorisation de molécules d'ARN ou d'ADN.

Le développement de systèmes performants pour administrer et transporter une molécule biologiquement active jusqu'à sa cible biologique constitue un enjeu majeur. En effet, aucun médicament ne peut exercer une activité thérapeutique si la substance active qu'il renferme n'est pas capable de franchir les barrières biologiques qui séparent le site d'administration du site d'action.

Les liposomes se sont désormais positionnés comme des candidats prometteurs dans ce domaine et leur introduction récente dans l'arsenal thérapeutique en cancérologie et en infectiologie représente l'aboutissement d'efforts considérables déployés en recherche et en développement.

Néanmoins, de nombreux problèmes demeurent notamment si l'on considère des modes d'administration par voie orale ou par voie sanguine. Ces problèmes sont liés à l'instabilité du liposome *in vivo* dans des milieux acides tels que par exemple celui observé dans le compartiment stomacal où règne un pH égal à 2, et/ou à la déstabilisation de la paroi liposomiale par interaction avec des protéines et lipoprotéines du sang.

Une approche possible pour améliorer les propriétés de ces formulations consiste à utiliser des lipides bipolaires d'archaebactéries thermophiles et méthanogènes apportant une stabilité accrue comparativement aux liposomes conventionnels. Pour cette raison, ceux-ci offrent un potentiel d'applications intéressant en tant que vecteurs de principes actifs et de gènes thérapeutiques ou comme systèmes d'administration de vaccins (WO 9722333; M. De Rosa et al., Thin Solid Films, 13-17, 284-285 (1996)).

Cependant, les techniques de culture, d'extraction et de purification utilisées actuellement ne permettent pas d'obtenir des quantités importantes de lipides naturels. La préparation de liposomes à partir de lipides synthétiques de structure parfaitement définie, s'avère donc être une alternative intéressante.

Un objet de la présente invention est de proposer des analogues de lipides membranaires d'archaebactéries dont la longueur de l'espaceur lipophile est comparable à celle des molécules naturelles.

Un autre objet de la présente invention est de décrire de nouvelles-compositions liposomiales intégrant de tels analogues.

Ces objectifs sont atteints grâce à l'invention, qui concerne des composés de formule générale (I) : 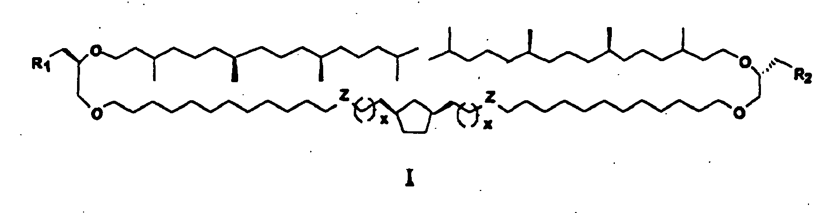 dans laquelle : . x est égal à zéro ou un ;
- Z représente un O, un S ou un CH₂ ;
- R₁ et R₂ qui peuvent être identiques ou différents, représentent l'un ou l'autre des substituants suivants :
- OH
- OY avec Y qui représente un groupement protecteur, préférentiellement un groupement allyle ou benzyle ou tétrahydropyranyle, ou trialkylsilyle ;
- OR₃, R₃ représentant un substituant monosaccharidique ou disaccharidique ;
- A₁-CH₂-N⁺(CH₃)₃, X⁻, X représentant un halogène, A₁ représente une liaison amide (NHC(O)) ou ester (OC(O)) ;
- OPO(OM)₂, M représentant un cation de métal alcalin ou alcalino-terreux ;
- OP(O)O⁻-O(CH₂)₂-N⁺(CH3)₃,
- A₂-(PEGₓ₁-A₃)ₙ-R₄, n étant égal à 0 ou 1, PEGₓ₁ étant un polyéthylèneglycol de poids moléculaire X₁, X₁ étant inférieur ou égal à 5000 daltons, A₂ et A₃ pouvant être identiques ou différents et représentant une liaison éther (O), ester (OC(O)), amide (NHC(O)), urée (NHC(O)NH), thiourée (NHC(S)NH), ou thioéther (S), **R₄** représentant un agent de ciblage.

Les composés hémimacrocycliques de formule générale (I) selon la présente invention sont caractérisés par :
- un squelette lipophile hémi-macrocyclique comprenant un espaceur incluant un motif cyclopentane 1,3-disubstitué au milieu de la chaîne lipophile et deux chaînes ramifiées dérivées du phytanol dont la longueur cumulée correspond à celle de l'espaceur et
- deux têtes polaires identiques ou différentes, neutres, anioniques, cationiques ou zwittérioniques.

Les composés selon la présente invention se caractérisent notamment par la présence inédite d'un motif cyclopentane dans leur formule.

Préférentiellement, lorsque les composés selon la présente invention intègrent un agent de ciblage, celui-ci est avantageusement choisi dans le groupe constitué par l'acide folique, les structures multiantennées comportant plusieurs motifs R₃, les anticorps, les peptides ; ces ligands étant reconnus spécifiquement par les récepteurs membranaires correspondants : récepteurs au folate pour l'acide folique, les intégrines pour les peptides RGD, les lectines pour les glycoconjugués.

Egalement préférentiellement, lorsque les composés selon la présente invention intègrent un substituant R₃, celui-ci est avantageusement choisi dans le groupe constitué par les substituants D-galactosyle, D-glucosyle, D-mannosyle, lactosyle, maltosyle.

Egalement préférentiellement, les composés selon la présente invention répondent à la formule générale (I) dans laquelle :
- x est égal à zéro ou un ;
- Z représente un O ou un CH₂ ;
- R₁ et R₂ sont identiques ou différents et représentent l'un ou l'autre des substituants suivants :
- OH
- OR₃, R₃ représentant un substituant lactosyle ;
- OP(O)O⁻O(CH₂)₂-N⁺(CH₃)₃;
- OPO(OM)₂, M représentant un cation de métal alcalin ou alcalino-terreux ;
- A₁-CH₂-N⁺(CH₃)₃, X⁻, X représentant un halogène, A₁ représente une liaison amide (NHC(O)) ou ester (OC(O)).

Selon une variante intéressante de l'invention, les composés selon celle-ci présentent des substituants **R₁** et **R₂** identiques et égaux à OR₃, R₃ étant un β-lactosyle.

Selon une autre variante intéressante de l'invention, les composés selon celle-ci présentent des substituants **R₁** et **R₂** identiques et égaux à OP(O)O⁻-O(CH₂)₂-N⁺(CH₃)₃.

Selon encore une autre variante intéressante de l'invention, les composés selon celle-ci présentent des substituants **R₁** et **R₂** identiques et égaux à NHC(O)CH₂-N⁺(CH₃)₃, X⁻, X représentant un halogène.

La présente invention couvre notamment les diols de formules (1) et (2)

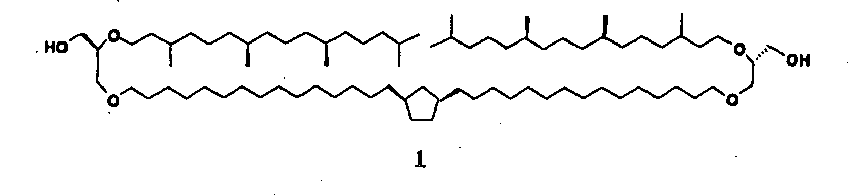

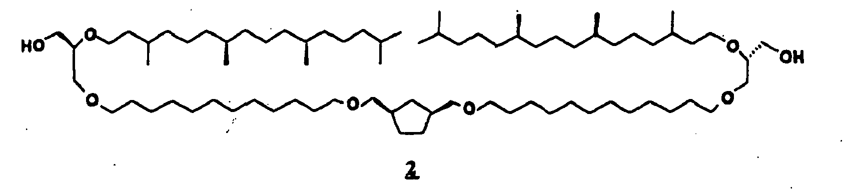

La présente invention couvre aussi les espaceurs de formules (3) et (4) utilisables pour la synthèse des composés décrits ci-dessus.

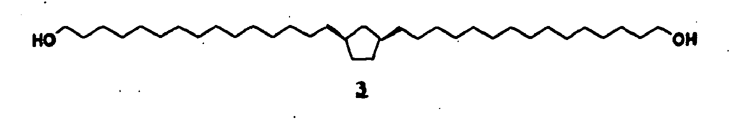

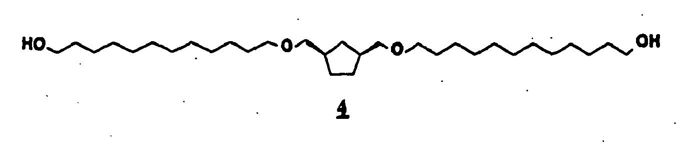

Cette synthèse pourra être effectuée par différentes voies connues de l'homme de l'art

En ce qui concerne les composés dans lesquels Z est égal à CH₂ ou à O cette synthèse peut comprendre une première étape consistant à coupler les espaceurs de formules **(3)** et **(4)** et le synthon chiral de formule **(5)** 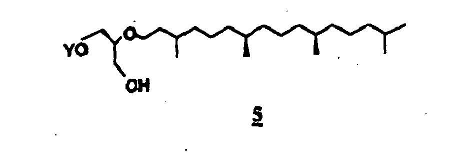 dans laquelle Y représente un groupe protecteur de type benzyle, allyle, tétrahydropyranyle ou trialkylsilyle.

L'espaceur de formule (3) peut être préparé selon un procédé qui consiste à créer deux doubles liaisons C=C simultanées par une double réaction de Wittig en un seul pot impliquant le diiodure de phosphonium de formule **(6)** 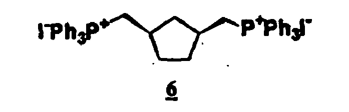 et l'aldéhyde de formule (7) 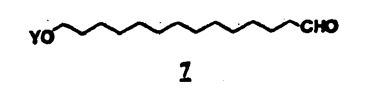 dans laquelle Y représente un groupe protecteur de type benzyle, allyle, tétrahydropyranyle ou trialkylsilyle.

Le diiodure de phosphonium de formule (6) est préparé en deux étapes à partir du *cis*-1,3-bis(hydroxyméthyl)cyclopentane de formule **(8)**

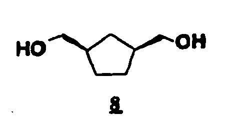

*via* une réaction de iodation suivie d'une substitution nucléophile par la triphénylphosphine.

Après l'étape de double réaction de Wittig, la déprotection des groupements hydroxyles et la réduction des doubles liaisons formées permettent de conduire à l'espaceur de formule (3).

L'espaceur de formule (4) peut être préparé selon un procédé qui consiste à réaliser une double alkylation entre le triflate (ou le mésylate, le paratoluène sulfonate ou les halogénures correspondants) de formule (9) et le diol **(8)** 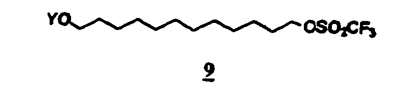 dans laquelle : Y représente un groupe protecteur de type benzyle, allyle, tétrahydropyranyle ou trialkylsilyle.

Le triflate de formule (9) est préparé par action de l'anhydride triflique en présence de 2,6-lutidine à partir de l'alcool de formule (10) dans laquelle Y représente un groupe protecteur de type benzyle, allyle, tétrahydropyranyle ou trialkylsilyle.

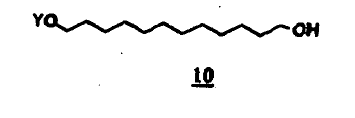

La réaction de di-*O*-alkylation entre le triflate **(9)** et le diol **(8)** s'effectue dans le dichlorométhane à reflux en présence de 1,8-bis-(diméthylamino)-naphtalène. Après la di-*O*-alkylation, l'élimination des groupements protecteurs Y permet d'obtenir l'espaceur de formule **(3).**

Après réaction des espaceurs (3) et (4) avec le synthon de formule **(5),** les groupements protecteurs Y sont éliminés pour conduire aux diols (1) et **(2).**

La dernière étape consiste ensuite, le cas échéant, à greffer un ou deux groupements hydrophiles de façon simultanée ou séquentielle selon le caractère recherché symétrique (R1=R2) ou dissymétrique (R1 différent de R2) des composés de formule générale (I).

L'accès aux composés symétriques β-bislactosylé dans lesquels **R1** et **R2** sont identiques et égaux à OR₃, R₃ étant un β-lactosyle repose sur une réaction de bisglycosylation des diols de formules (2) et (3) à partir du thiolactosyle peracétylé de formule (11): 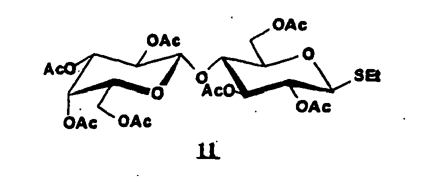 dans des conditions standard d'activation (*N*-iodosuccinimide (NIS), triflate de triéthylsilyle (Et₃SiOTf), dichlorométhane), suivie par une désacétylation des hydroxyles du disaccharide selon la procédure de Zemplèn (CH₃ONa, CH₃OH).

L'accès aux composés dans lesquels **R1** et **R2** sont identiques et égaux à OR₃, R₃ étant un substituant mannosyle ou galactosyle sont préparés selon des conditions similaires.

Les composés symétriques dans lesquels **R1** et **R2** sont des phosphatidylcholines (R₁=R₂= OP(O)O⁻-O(CH₂)₂-N⁺(CH₃)₃) sont obtenus en deux étapes par réaction entre le bromoéthyldichlorophosphate Cl₂P(O)O-(CH₂)₂-Br et les diols de formules (2) et (3) en présence de triéthylamine, suivie de la réaction de la triméthylamine avec les dérivés bromophosphates ainsi obtenus (R₁=R₂= OP(O)O⁻-(CH₂)₂-Br).

Les composés de formule générale (I) symétriques dans lesquels **R1** et **R2** sont des bétaïnes (R1=R2= NHC(O)CH₂-N⁺(CH₃)₃, X⁻, X représentant un halogène), reliés au segment lipophile *via* préférentiellement des liaisons de type amide, sont obtenus par couplage entre la diamine **(12)** et la glycine bétaïne sous forme activée (chlorure d'acyle **(13)** ou thiazolidine-**2**-thione (14)) en présence de triéthylamine dans le dichlorométhane.

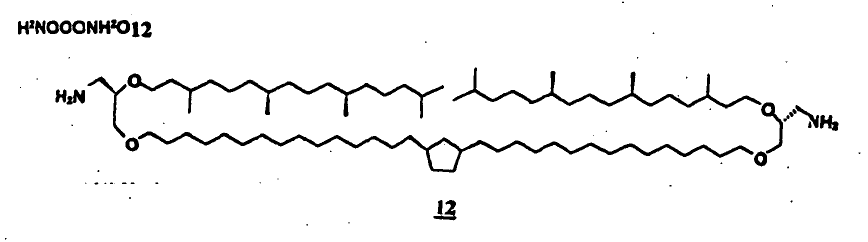

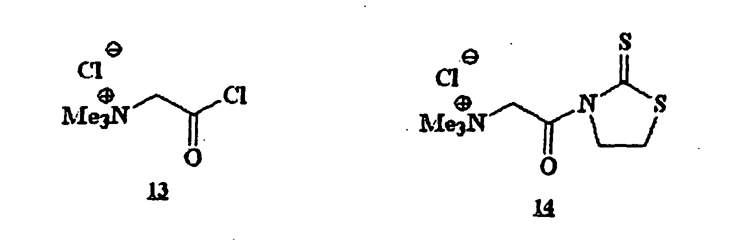

Les composés de formule générale (I) disymétriques comportant deux têtes polaires R1 et R2 différentes sont préparés selon un procédé qui consiste à désymétriser les diols de formules (1) et (2) par l'introduction d'un groupement benzylique (ou un autre groupement protecteur analogue) pour conduire aux alcools de formules **(15)** et **(16) :**

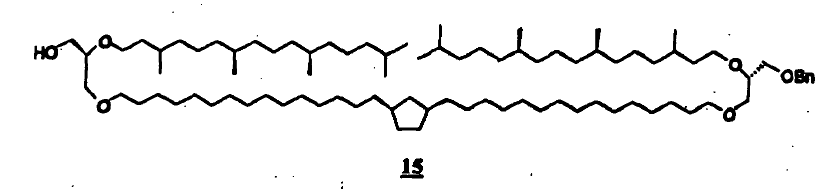

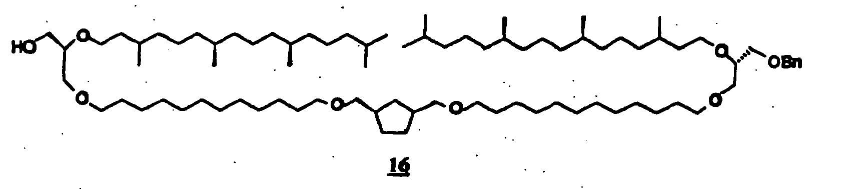

Cette mono-protection des diols de formules (1) et (2) est réalisée préférentiellement par action du bromure de benzyle (BnBr) en présence d'oxyde d'argent (Ag₂O) dans le dichlorométhane. A ce stade, une première tête polaire est introduite au niveau de l'hydroxyle libre, puis avec hydrogénolyse, la seconde tête est incorporée. L'élimination finale des groupes protecteurs présents au niveau des têtes polaires permet de conduire aux structures dissymétriques cibles.

L'invention concerne également des compositions liposomiales incorporant au moins un composé de formule générale (I) selon l'invention décrits ci-dessus seul(s) ou en mélange avec un ou plusieurs colipide(s) synthétique(s) ou naturel(s).

Les composés selon l'invention permettent de conférer à de telles compositions liposomiales, une plus grande stabilité, notamment en milieu acide et vis-à-vis des protéines et lipoprotéines du sang.

L'invention concerne notamment mais, non exclusivement, de telles compositions liposomiales dans lesquels le colipide est de la phosphatidylcholine de lécithine d'oeuf.

L'invention concerne aussi de telles compositions liposomiales contenant au moins un composé de formule générale (I) dans laquelle **R₁** et **R₂** sont identiques ou différents et représentent l'un et/ou l'autre des motifs suivants :
- A₁-CH₂-N-(CH₃)₃, X⁻, X représentant un halogène, A₁ représente une liaison amide (NHC(O)) ou ester (OC(O)) ;
- A₂-(PEGₓ₁,-A₃)ₙ-R₄, n étant égal à 0 ou 1, PEGₓ₁, étant un polyéthylèneglycol de poids moléculaire X₁, X₁ étant inférieur ou égal à 5000 daltons, A₂ et A₃ pouvant être identiques ou différents et représentant une liaison éther (O), ester (OC(O)), amide (NHC(O)), urée (NHC(O)NH), thiourée (NHC(S)NH), ou thioéther (S), **R₄** représentant un agent de ciblage ;
   et au moins un co-lipide cationique
   et au moins un co-lipide fusogène.

Le co-lipide cationique pourra par exemple être un lipide cationique bicaténaire de formule MM12 ou MM16 (J.Gene Med ; 2002 ;4 ;415-427):

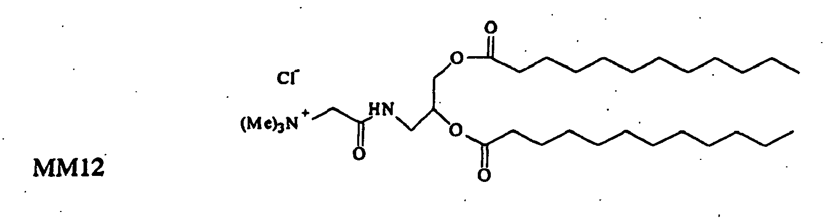

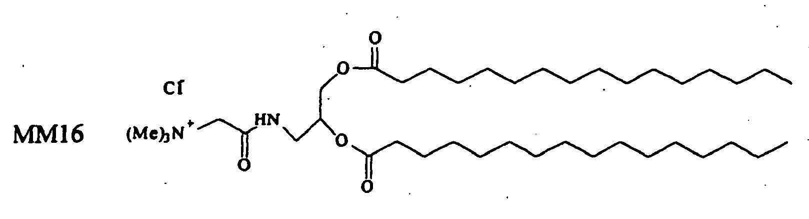

Le co-lipide fusogène pourra par exemple être la dioléylphosphatidyléthanolamine (DOPE) ou le cholestérol.

La présente invention concerne également l'utilisation des compositions liposomiales décrites ci-dessus pour la vectorisaton, c'est-à-dire le transfert transmembranaire de molécules d'intérêt thérapeutique et/ou de molécules d'ADN ou d'ARN.

Notamment l'invention concerne l'utilisation de ces compositions liposomiales pour le transfert transmembranaire de gènes.

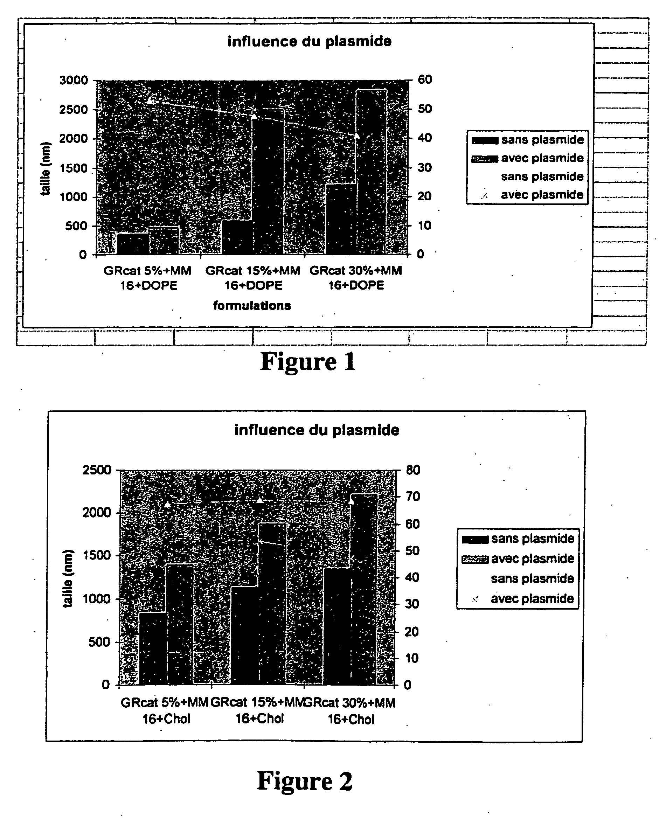

L'invention sera plus facilement comprise grâce à la description qui va suivre d'exemples de réalisation donnés en référence aux dessins dans lesquels les figures 1 et 2 représentent l'influence du plasmide (pCMVLuc) sur le potentiel zêta et la taille de différents complexes intégrant des compositions liposomiales selon l'invention.

### Exemple 1 : espaceur de formule (4)

Le diol (8) (500 mg ; 3.85 mmol) est dissout dans 50 mL de CH₂Cl₂, anhydre et le proton sponge (1.7 g ; 2.3 éq) y est rajouté. Le milieu est agité pendant une heure à température ambiante. Ensuite le triflate (9) est ajouté lentement (4.4 g ; 2.6 éq) et le mélange est porté à reflux pendant 2 jours. Après refroidissement, le brut réactionnel est filtré pour éliminer les sels de proton sponge. Le CH₂Cl₂ est concentré sous vide.

Le produit dibenzylé obtenu est purifié par colonne sur gel de silice (éluant : éther de pétrole/acétate d'éthyle 95/5 v/v) et 1.92 g de solide blanc sont récupérés (rendement : 74%).

Ce produit dibenzylé ainsi préparé (2,6 g ; 4mmol) est solubilisé dans 100 mL de cyclohexane et le palladium acétate est ajouté (150 mg ; 5% en masse). Le milieu est placé sous atmosphère d'hydrogène et agité pendant 5 heures. Ensuite le milieu est chauffé jusqu'à dissolution totale du produit et filtré à chaud sur célite et concentré sous vide. Le solide est solubilisé à chaud dans du cyclohexane ; après refroidissement le produit est récupéré par filtration sur Büchner. On récupère ainsi 1,7 g du composé (4) qui se présente sous la forme d'un solide blanc (rendement : 90%).

### Exemple 2: espacer de formule (3)

A une suspension du sel de phosphonium (6) (300 mg ; 0,34 mmol) dans 6 mL de THF anhydre, on ajoute, à 0°C, le butyllithium en solution dans l'hexane (2M) (360 µL ; 0,72 mmol ; 2,1 éq) ; une coloration orangée apparaît. Le milieu réactionnel est agité pendant 15 minutes à 0°C, puis l'aldéhyde (7) (Y est un benzyl) (239 mg ; 0,69 mmol ; 2,0 éq) en solution dans 6 mL de THF anhydre est canulé. Une décoloration progressive est observée ainsi que la formation d'un précipité. Après quelques minutes, l'excès de butyllithium est détruit par de l'eau. Le mélange est extrait trois fois avec un mélange EP/AcOEt (6/4 v/v) puis les phases organiques sont lavées à l'eau, séchées, filtrées et concentrées. Une chromatographie sur colonne de gel de silice (25 éq, éluant : EP/AcOEt (99/1 v/v)) permet d'isoler 150 mg d'espaceur insaturé (0,21 mmol) se présentant sous la forme d'un solide blanc (rendement : 63%).

Le produit benzylé (1,93 g ; 2,8 mmol) est solubilisé dans l'éthanol (150 mL) puis additionné de Palladium sur charbon actif (500 mg). Le mélange réactionnel est placé sous atmosphère d'hydrogène pendant une nuit. Après avoir légèrement chauffé le milieu réactionnel, celui-ci est filtré à chaud sur célite, le solvant est évaporé. On obtient 1,34 g (2,6 mmol) de produit (3) qui se présente sour la forme d'un solide blanc (rendement : 93 %)

### Exemple 3 : composé de formule (2)

Dans un ballon sec sont introduits de la 2,6-lutidine (425 µL ; 3,8 éq) puis 20 mL de dichlorométhane anhydre. A 0°C, l'anhydride triflique (600 µL ; 3,8 éq) est ajouté et le mélange est agité 15 minutes à température ambiante. Le diol (4) (500 mg ; 1 mmol) sous la forme solide est additionné en une seule fois. Après quelques minutes, de l'eau est ajoutée, la phase aqueuse est extraite 3 fois au dichlorométhane. Les phases organiques sont rassemblées, lavées avec une solution aqueuse à 5% d'acide chlorhydrique, puis une solution aqueuse à 5% d'hydrogénocarbonate de sodium et enfin avec une solution saturée de chlorure de sodium. La phase organique est séchée sur MgSO₄, filtrée, et concentrée. Le produit est purifié rapidement, car instable, par chromatographie sur colonne de gel de silice (éluant : EP/AcOEt (8/2 v/v)). Le ditriflate ainsi obtenu (Rf 0,8 (EP/AcOEt 8/2 v/v)) est mis immédiatement en réaction.

A une suspension d'hydrure de potassium (568 mg ; 4 éq), en suspension dans 6 mL de THF anhydre on additionne, à 0°C, l'alcool (5) (Y est un benzyl) (1,5 g ; 3 éq) dissous dans 6 mL de THF anhydre. Le mélange réactionnel est agité 30 minutes à température ambiante. Le triflate (800 mg ; 1.05 mmol ; 1 éq) dissous dans 8 mL de THF anhydre est ajouté goutte à goutte. Après 2 heures, l'excès d'hydrure de potassium est détruit par de l'eau. Une extraction à l'éther éthylique est réalisée. La phase organique est séchée sur sulfate de magnésium puis filtrée et concentrée. Une purification par chromatographie sur colonne de gel de silice (40 éq, éluant : EP/AcOEt (99/1 v/v)) permet d'isoler 23 mg (0,45 mmol) du composé benzylé qui se présente sous la forme d'une huile incolore (rendement 45% (sur les deux étapes)).

Le produit ainsi préparé (623 mg ; 0,45 mmol) est solubilisé dans 10 mL d'acétate d'éthyle et le palladium acétate est ajouté (30 mg ; 5% en masse). Le milieu est placé sous atmosphère d'hydrogène et agité pendant 24 heures. Le milieu réactionnel est ensuite filtré sur célite et concentré sous vide. On récupère ainsi 463 mg du composé (2) qui se présente également sous la forme d'une huile incolore (Rendement : 85%).

### Exemple 4: composé de formule (1)

Dans un ballon bien sec on introduit la 2,6-lutidine (423 µL ; 3,60 mmol ; 3,8 éq), 20 mL de dichlorométhane anhydre et à 0°C, l'anhydride triflique (611 µL ; 3,60 mmol ; 3,8 éq). Après 10 minutes d'agitation à 0°C, le diol (3) (500 mg ; 0,96 mmol) sous la forme solide est additionné en une seule fois. Le mélange réactionnel est remonté à température ambiante, puis chauffé à 30°C ; le diol se dissout. Après quelques minutes, après addition d'eau, la phase aqueuse est extraite 3 fois au dichlorométhane. Les phases organiques sont rassemblées, lavées avec une solution aqueuse à 5% d'acide chlorhydrique, puis une solution aqueuse d'hydrogénocarbonate de sodium 5% et enfin avec une solution saturée de chlorure de sodium. La phase organique est séchée, filtrée, et concentrée. Le produit est purifié rapidement par chromatographie sur colonne de gel de silice (20 éq, éluant : EP/AcOEt (9/1 v/v)). Le triflate ainsi obtenu, qui se présente sous la forme d'un solide blanc, est mis immédiatement en réaction.

A 0°C, le triflate précédemment décrit (750 mg ; 0,96 mmol ; 1 éq) dans 6 mL de THF anhydre est ajouté goutte à goutte à une suspension d'hydrure de potassium (438 mg ; 3,8 mmol ; 4 éq) et d'alcool 5 (1,32 g ; 2,87 mmol ; 3 éq) dans 12 mL de THF anhydre. Le mélange réactionnel est agité 10 minutes à 0°C. Après quelques minutes, après addition d'eau, une extraction à l'éther diéthylique est réalisée. La phase organique est séchée sur sulfate de magnésium puis filtrée et concentrée. Une purification par chromatographie sur colonne de gel de silice (40 éq, éluant : EP/AcOEt (1/0 puis 95/5 v/v)) permet d'isoler le produit pseudo-macrocyclique benzylé (860 mg ; 0,61 mmol) qui se présente sous la forme d'une huile jaunâtre (rendement : 64 % (2 étapes)).

Le diol hémi-macrocyclique dibenzylé (1,77 g ; 1,2 mmol), solubilisé dans de l'acétate d'éthyle (50 mL), est mis sous atmosphère d'hydrogène pendant une nuit, en présence de palladium sur charbon actif (500 mg). Une filtration sur célite, à chaud est réalisée et le composé (1) (1,2 g ; 0,97 mmol), qui se présente sous la forme d'une huile, est isolé après concentration sous pression réduite (rendement : 80%).

### Exemple 5 : Compositions liposomiales

Différentes compositions liposomiales B, C , D, E, E1 ont été préparées par hydratation d'un film lipidique par une solution contenant de la carboxyfluorescéine à 2,5%. Après hydratation des films lipidiques constitués d'un mélange de lipides ou de lipides synthétiques purs, puis agitation de la solution pendant quelques heures, les échantillons ont été extrudés à travers une membrane en polycarbonate (400nm puis 200nm). Une filtration sur gel Sephadex G75 a permis de recueillir les compositions liposomiales.

La stabilité de ces différentes compositions liposomiales selon l'invention a été testé à une température de 37°C:
en présence d'un composé tensio-actif (solution aqueuse de cholate de sodium à 0.4%) afin de modéliser le comportement de ces compositions en présence de sels biliaires (test n° 1) ;
en présence de sérum de veau riche en lipoprotéines afin de modéliser le comportement de ces compositions dans le milieu sanguin (test n°3) ;
en milieu acide, à pH 2-3 (tampon 1XKRB), afin de mimer le comportement de ces compositions dans le compartiment stomacal (test n° 3).

Ces tests reposent sur la détermination par spectrofluorimétrie du taux de relargage d'une sonde fluorescente encapsulée. Dans les deux premiers cas, on mesure par spectrofluorimétrie le relargage de la sonde fluorescente. Pour l'étude en fonction du pH, après incubation, le pH est neutralisé et on effectue une dialyse de la carboxyfluorescéine libre. Les liposomes sont alors lysés et on dose la carboxyfluorescéine qui était restée encapsulée.

Les résultats obtenus sont donnés dans les tableaux 1 à 3 ci-après en référence à une composition liposomiale A ne contenant que de la phosphatidylcholine de lécithine d'oeuf (EPC).

### Résultats du test n°1 (en présence du tensio actif),

Le tableau 1 ci-après donne des résultats obtenus dans le cadre du test n°1 avec différentes compositions liposomiales.

**Tableau 1**

| Composition liposomiale | Composé selon l'invention | teneur (massique) | Co-lipide | teneur (massique) | % de relargage |
|---|---|---|---|---|---|
| A | Aucun | 0 | EPC* | 100 | 100 |
| B | de formule 1 | 20 | EPC | 80 | 50 |
| C | de formule 1 | 30 | EPC | 70 | 30 |
| D | De formule avec x=1 Z=CH₂ et R1=R2=β-lactosyle | I 60 | EPC | 40 | 40 |

Ces résultats montrent que les composés de la présente invention indiqués dans la deuxième colonne de ce tableau 1 apportent aux compositions liposomiales B, C, D un gain de stabilité en présence d'un composé tensio-actif, ce qui tend à prouver que les compositions selon l'invention présenteront une stabilité accrue en présence des sels biliaires.

Il a par ailleurs été observé que ces composées synthétiques incorporés dans certaines formulations, induisent une meilleure stabilité que les diols macrocycliques naturels (diglycéroltétraéther -DGTE) obtenus après hydrolyse des têtes polaires.

### Résultats du test n°2 (en présence de sérum de veau).

Le tableau 2 ci-après donne des résultats obtenus dans le cadre du test n°2 avec différentes compositions liposomiales.

**Tableau 2**

| Composition liposomiale | Composé selon l'invention | Teneur (massique) | Co-lipide* | Teneur (massique) | % de relargage |
|---|---|---|---|---|---|
| A | Aucun | 0 | EPC* | 100 | 100 |
| E | De formule I avec x=1 Z=CH₂ et R1=R2=phosphatidyl choline | 100 | Aucun | | 30 |
| E1 | De formule I avec x=1 Z=CH₂ et R1=R2=phosphatidyl choline | 60 | EPC | 40 | 60 |
| D | De formule I avec x=1 Z=CH₂ et R1=R2=β-lactosyl | 60 | EPC | 40 | 40 |

Ces résultats montrent que les composés de la présente invention indiqués dans la deuxième colonne de ce tableau 2 apportent aux compositions liposomiales D, E, E1 un gain de stabilité en présence de sérum de veau, riche en lipoprotéines, ce qui tend à prouver que les compositions selon l'invention présenteront une stabilité accrue en milieu sanguin.

### Résultats du test n°3 (en milieu acide).

Le tableau 3 ci-après donne des résultats obtenus dans le cadre du test n°3 avec différentes compositions liposomiales.

**Tableau 3**

| Composition liposomiale | Composé selon l'invention | Teneur (massique) | Co-lipide* | Teneur (massique) | % de relargage ** |
|---|---|---|---|---|---|
| A | Aucun | 0 | EPC* | 100 | 95 |
| E | De formule I avec x=1 Z=CH₂ et R1=R2=phosphatidyl choline | 100 | aucun | | 30 |
| E1 | De formule I avec x=1 Z=CH₂ et R1=R2=phosphatidyl choline | 60 | EPC | 40 | 85 |

| | | | | | |
|---|---|---|---|---|---|
| ** après 10 minutes d'incubation. | | | | | |

Ces résultats montrent que les composés de la présente invention indiqués dans la deuxième colonne de ce tableau 3 apportent aux compositions liposomiales un gain de stabilité en pH acide, ce qui tend à prouver que les compositions selon l'invention présenteront une stabilité accrue dans le compartiment stomacal. Cette stabilité est comparable (20-30% de relargage) à celle obtenue avec des lipides naturels extraits de *Thermoplasma acidophilium* (espèce thermoacidophile qui possède une double tolérance aux températures élevées et aux pH faibles)

### Exemple 6 : Vectorisation

Différentes compositions liposomiales selon la présente invention ont également été réalisées afin de tester leur aptitude à vectoriser le plasmide pCMVLuc.

Le composé de formule générale (I) utilisé dans ces compositions est celui dans lequel x=0 ou 1 Z=O ou CH₂ et dans lequel R₁=R₂=NHC(O)CH₂N⁺Me₃,Cl. Ce composé est ci-après dénommé GRcat.

Ce composé a été associé à un mélange de co-lipides constitués à parts égales de dioléylphosphatidyléthanolamine (DOPE) ou de cholestérol (Chol) d'une part et de lipide cationique bicaténaire MM16 d'autre part.

Le détail de ces compositions liposomiales est donné dans le tableau 4 ci-après.

**Tableau 4**

| Composition | Composé selon l'invention | Teneur (massique) | Co-lipides | Teneur (massique) |
|---|---|---|---|---|
| F1 | GRcat | 5% | DOPE +MM16 | 95 % |
| F2 | GRcat | 15% | DOPE +MM16 | 85 % |
| F3 | GRcat | 30% | DOPE +MM16 | 70 % |
| F4 | GRcat | 5% | Chol +MM16 | 95% |
| F5 | GRcat | 15% | Chol +MM16 | 85% |
| F6 | GRcat | 30% | Chol +MM16 | 70% |

Des mesures de diffusion dynamique de la lumière (détermination de la taille des objets) et de potentiel zêta (évaluation de la charge globale des objets) ont par ailleurs permis de déterminer la taille et le rapport de charge global de ces différentes compositions liposomiales avant et après ajout du plasmide pCMV Luc.

Les résultats obtenus sont portés sur les figures 1 et 2 jointes.

Ces résultats mettent en évidence la formation d'agrégats supramoléculaires issus de l'interaction électrostatique entre les vésicules cationiques et le plasmide (pCMV Luc).

En effet, après ajout du plasmide, on observe une augmentation de la taille des objets avec en parallèle une diminution du rapport de charge global qui reste néanmoins positif ; un potentiel zêta positif après complexation avec le plasmide étant nécessaire dans le cadre d'une vectorisation sans agent de ciblage car l'internalisation des agrégats se fait par interactions électrostatiques entre la membrane cellulaire (chargée négativement) et la vésicule (chargée positivement).

Il apparaît qu'une augmentation du taux d'incorporation du composé GRcat selon la présente invention entraîne une augmentation de la taille des liposomes et une augmentation (encore plus importante) de la taille des complexes liposomes-ADN.

L'augmentation du taux d'incorporation n'entraîne pas d'effets importants sur la valeur du potentiel zêta, mais en revanche, la formation des agrégats supramoléculaires avec l'ADN entraîne une diminution du potentiel zêta.

Les compositions liposomiales selon la présente invention, dont on peut contrôler la taille et le potentiel zêta, constituent donc des systèmes susceptibles d'être utilisés pour la vectorisation de molécules d'ADN et d'ARN et notamment de gènes.

Les études de transfection *in vitro* menées à partir des formulations incorporant les tétraéthers I dicationiques (R1 = R2 = NH(CO)-CH₂-N⁺(CH₃)₃ ; x = 0 et Z = O ou x = 1 et Z = CH₂), et la DOPE ou le cholestérol comme co-lipide, pour 4 µg d'ADN délivré (plasmide pEGFP-N1 codant pour la protéine GFP sous le contrôle du promoteur du cytomégalovirus, pCMV), sur les cellules A549 (cellules épithéliales alvéolaires humaines de type II), à différents rapports de charges (R (+/-) = 0,5 ; 1 ; 2 ; 4 ; 8), ont montré que de faibles quantités de co-lipides (DOPE ou cholestérol) suffisent pour assurer une bonne efficacité de transfection.

En effet, les meilleurs résultats sont obtenus avec les formulations mettant en jeu le tétraéther 1 (R1 = R2 = NH(CO)-CH₂-N⁺(CH₃)₃ ; x = 0 et Z = O) comme lipide cationique en présence de DOPE. Une très forte efficacité (90% de cellules vivantes et transfectées) est obtenue pour 5% ou 15% de DOPE et pour R (+/-) =8.

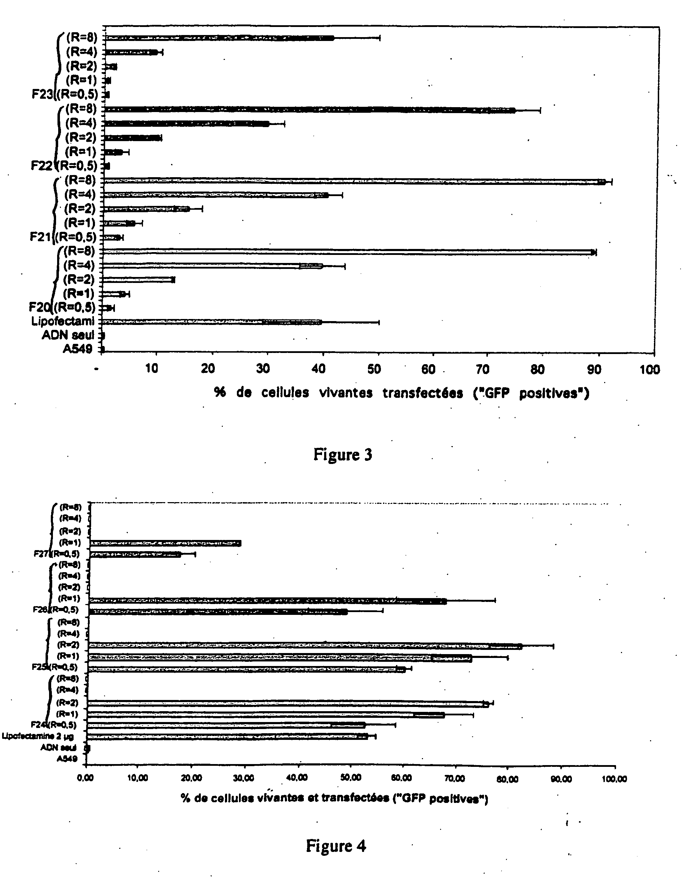

La figure 3 montre l'efficacité de transfection des formulations de type tétraéther I ( R1 = R2 = NH(CO)-CH₂-N⁺(CH₃)₃ ; x = 0 et Z = O)/ DOPE sur les cellules A549 pour 4 µg d'ADN délivré.

Sur cette figure « Lipofectami » correspond à la Lipofectamine (lipide cationique commercial de référence).

R représente le rapport de charges (+/-).

F20 indique 5% de DOPE. F21 15% de DOPE F22 30 % de DOPE et F23 50% de DOPE.

Dans le cas du tétraéther **I** (R1 = R2 = NH(CO)-CH₂-N⁺(CH₃)₃ ; x = 1 et Z = CH₂), l'efficacité maximale est observée à partir de formulations incorporant 15% de DOPE pour un rapport de charge R (+/-) inférieur à 4 (82% de cellules vivantes et transfectées). Lorsque le rapport de charges augmente, aucune efficacité n'est observée avec le composé **I** ( R1 = R2 = NH(CO)-CH₂-N⁺(CH₃)₃ ; x = 1 et Z = CH₂). Cette observation pourrait s'expliquer, dans ce cas, par une trop forte internalisation des complexes lipides/ADN. L'entrée d'une quantité importante de lipides dans les cellules entraînerait une déstabilisation des membranes cellulaires et par conséquent un fort taux de mortalité.

La figure 4 montre l'efficacité de transfection des formulations de type tétraéther I ( R1 = R2 = NH(CO)-CH₂-N⁺(CH₃)₃ ; x = 1 et Z = CH₂)/ DOPE sur les cellules A549 pour 4 µg d'ADN délivré.

La référence est également la Lipofectamine.

R représente le rapport de charges (+/-).

F24 indique 5% de DOPE. F25 15% de DOPE, F26 30 % de DOPE et F27 50% de DOPE.

Ces résultats démontrent clairement le fort potentiel de ces tétraéthers cationiques pour le transfert de gènes.

## Revendications

1. Composé de formule générale (I) : 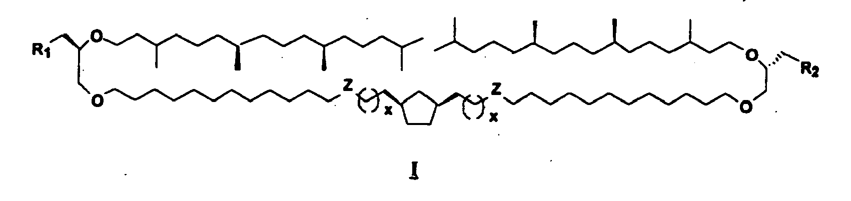 dans laquelle : . x est égal à zéro ou un ;
. Z représente un O, un S ou un CH₂ ;
. R₁ et R₂ qui peuvent être identiques ou différents, représentent l'un ou l'autre des substituants suivants :
. OH
. OY avec Y qui représente un groupement protecteur ;
. OR₃, R₃ représentant un substituant monosaccharide ou disaccharide ;
. A₁-CH₂-N⁺(CH₃)₃, X, X représentant un halogène, A₁ représente une liaison amide (NHC(O)) ou ester (OC(O)) ;
. OPO(OM)₂, M représentant un cation de métal alcalin ou alcalino-terreux ;
. OP(O)O⁻-O(CH₂)₂-N⁺(CH₃)₃.
. A₂-(PEGₓ₁,-A₃)ₙ-R₄, n étant égal à 0 ou 1, PEGₓ₁ étant un polyéthylèneglycol de poids moléculaire X₁, X₁ étant inférieur ou égal à 5000 daltons, A₂ et A₃ pouvant être identiques ou différents et représentant une liaison éther (O), ester (OC(O)), amide (NHC(O)), urée (NHC(O)NH), thiourée (NHC(S)NH), ou thioéther (S), R₄ représentant un agent de ciblage.

2. Composé selon la revendication 1 **caractérisé en ce que** ledit groupement protecteur est un groupement allyle ou benzyle ou tétrahydropyranyle ou trialkylsilyle.

3. Composé selon la revendication 1 ou 2 **caractérisé en ce que** ledit agent de ciblage est choisi dans le groupe constitué par l'acide folique, les structures multiantennées comportant plusieurs motifs R₃, les anticorps, les peptides.

4. Composé selon l'une des revendications 1 à 3 **caractérisé en ce que** R₃ est un substituant choisi dans le groupe constitué par les substituants D-galactosyle, D-glucosyle, D-mannosyle, lactosyle, maltosyle.

5. Composé selon l'une quelconque des revendications 1 ou 4 répondant à la formule (I) dans laquelle :
. x est égal à zéro ou un ;
. Z représente un O ou un CH₂ ;
. R₁ et R₂ sont identiques ou différents et représentent l'un ou l'autre des substituants suivants :
. OH
. OR₃, R₃ représentant un lactosyle ;
. OP(O)O⁻-O(CH₂)₂-N⁺(CH₃)₃ ;
. OPO(OM)₂, M représentant un cation de métal alcalin ou alcalino-terreux ;
. A₁-CH₂-N⁺(CH₃)₃, X⁻, X représentant un halogène, A₁ représentant une liaison amide (NHC(O)) ou ester (OC(O)).

6. Composé selon la revendication 5 **caractérisé en ce qu'**il répond à la formule (I) dans laquelle R₁=R₂=OR₃, R₃=β-lactosyle

7. Composé selon la revendication 1 **caractérisé en ce qu'**il répond à la formule (I) dans laquelle R₁=R₂= OP(O)O⁻-O(CH₂)₂-N⁺(CH₃),

8. Composé selon la revendication 1 **caractérisé en ce qu'**il répond à la formule (I) dans laquelle R1=R2= NHC(O)CH₂-N⁺(CH₃)₃, X⁻, X représentant un halogène.

9. Composé selon la revendication 8 **caractérisé en ce que** X est le chlore et le brome.

10. Composé selon la revendication 1 **caractérisé en ce qu'**il répond à la formule (1) :

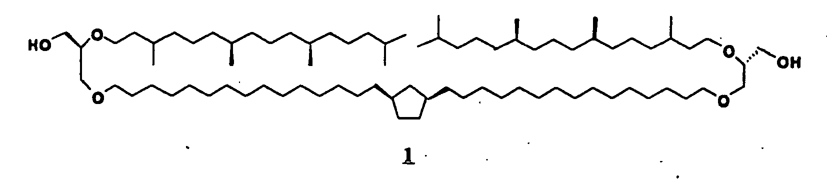

11. Composé selon la revendication 1 **caractérisé en ce qu'**il répond à la formule (2) :

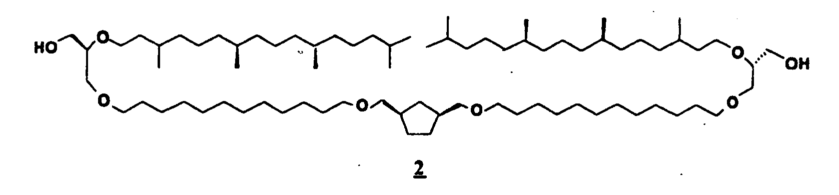

12. Composé pour la synthèse du composé selon la formula. (1) **caractérisé en ce qu'**il répond à la formule (3)

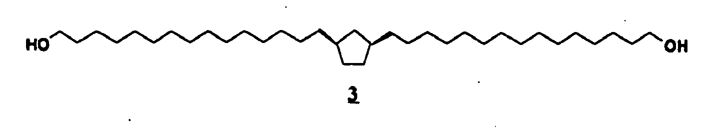

13. Composé pour la synthèse du composé selon la formule (2) **caractérisé en ce qu'**il répond à la formule (4)

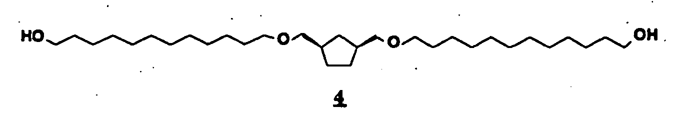

14. Composition liposomiale incorporant au moins un composé de formule générale (I) selon l'une des revendications 1 à 11.

15. Composition selon la revendication 14 **caractérisée en ce qu'**elle inclut au moins un co-lipide.

16. Composition selon la revendication 15 **caractérisé en ce que** ledit colipide est la phosphatidylcholine de lécithine d'oeuf.

17. Composition selon la revendication 15 ou 16 **caractérisée en ce qu'**elle comprend un composé de formule générale I dans laquelle R₁ et R₂ sont identiques ou différents et représentent l'un et/ou l'autre des motifs suivants :
. A₁-CH₂-N⁺(CH₃)₃, X⁻, X représentant un halogène, A₁ représente une liaison amide (NHC(O)) ou ester (OC(O)) ;
.. A₂-(PEGₓ₁-A₃)ₙ-R₄, n étant égal à 0 ou 1, PEGₓ₁, étant un polyéthylèneglycol de poids moléculaire X₁, X₁ étant inférieur ou égal à 5000 daltons, A₂ et A₃ pouvant être identiques ou différents et représentant une liaison éther (O), ester (OC(O)), amide (NHC(O)), urée (NHC(O)NH), thiourée (NHC(S)NH), ou thioéther (S), R₄ représentant un agent de ciblage ;
et au moins un co-lipide cationique
et au moins un co-lipide fusogène.

18. Composition selon la revendication 17 **caractérisé en ce que** ledit co-lipide cationique est un lipide cationique bicaténaire MM12 ou MM16.

19. Composition selon la revendication 17 ou la revendication 18 **caractérisé en ce que** ledit co-lipide fusogène est la dioléylphosphatidyléthanolamine ou le cholestérol.

20. Utilisation d'une composition liposomiale selon l'une quelconque des revendications 14 à 19 pour la vectorisation de molécules d'intérêt thérapeutique.

21. Utilisation d'une composition liposomiale selon l'une quelconque des revendications 14 à 19 pour la vectorisation de molécules d'ADN ou d'ARN.

## Claims

1. Compound with the general formula (I):
in which: 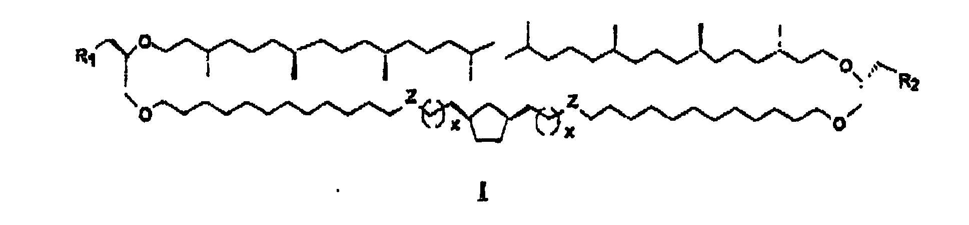
- X is equal to zero or one;
- Z represents an O, an S or a CH₂,
- R₁ and R₂, which can be identical or different, represent one of the following substituents:
- OH
- OY with Y representing a protector group;
- OR₃, R₃ representing a monosaccharide or disaccharide substituent;
- A₁-CH₂-N⁺(CH₃) ₃, X⁻, X representing an halogen, A₁ representing an amide (NHC(O)) or ester (OC(O))bond;
- OPO(OM)₂, M representing an alkaline metal cation or alkaline-earth metal;
- OP (O) O⁻-O (CH₂) ₂-N⁺ (CH₃) ₃,
- A₂-(PEG_{X1}-A₃)ₙ-R₄, n being equal to 0 or 1, PEG_{X1} being a polyethyleneglycol of molecular weight X₁, X₁ being less than or equal to 5,000 daltons, A₂ and A₃
being identical or different and representing one ether (O), ester (OC(O)), amide (NHC(O)), urea (NHC(O)NH), thiourea (NHC(S)NH), or thioether (S) bond, R₄ representing a targeting agent.

2. The compound according to claim 1 **characterised**
**in that** said protector group is an allyl, benzyl, tetrahydropyranyl, or trialkylsilyl group.

3. The compound according to claim 1 or 2
**characterised in that** said targeting agent is chosen from the group constituted of the folic acid, multi-antenna structures comprising several R₃ motifs, antibodies, and peptides.

4. The compound according to claims 1 to 3
**characterised in that** R₃ is a substituent chosen from the group constituted of the D-galactosyl, D-glucosyl, D-mannosyl, lactosyl, and maltosyl substituents.

5. The compound according to any one of claims 1 or 4 corresponding to formula (I) in which:
- x is equal to zero or one;
- Z represents an O or a CH₂;
- R₁ and R₂ are identical or different and represent one of the following substituents:
- OH
- OR₃, R₃ representing a lactosyl;
- OP(O)O⁻-O (CH₂)₂-N⁺(CH₃)₃;
- OPO(OM)₂, M representing an alkaline metal cation or alkaline-earth metal;
- A₁-CH₂-N⁺ (CH₃) ₃, X⁻, X representing an halogen, A₁ representing an amide (NHC(O)) or ester (OC(O)) bond.

6. The compound according to claim 5 **characterised**
**in that** it corresponds to formula (I) in which R₁=R₂=OR₃, R₃=β-lactosyl.

7. The compound according to claim 1 **characterised**
**in that** it corresponds to formula (I) in which R₁=R₂= OP (O)O⁻-O(CH₂)₂-N⁺(CH₃)₃.

8. The compound according to claim 1 **characterised**
**in that** it corresponds to formula (I) in which R₁=R₂= NHC (O)CH₂-N⁺(CH₃)₃, X⁻, X represent an halogen.

9. The compound according to claim 8 **characterised**
**in that** X is chlorine and bromine.

10. The compound according to claim 1
**characterised in that** it corresponds to formula (1):

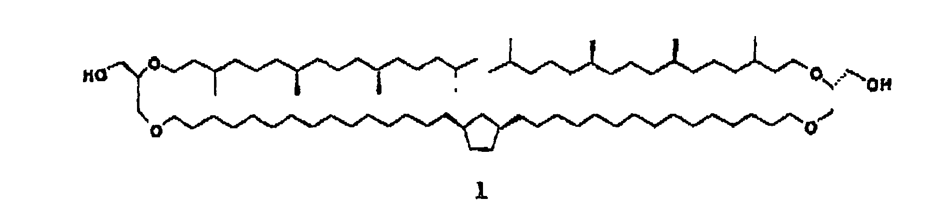

11. The compound according to claim 1
**characterised in that** it corresponds to formula (2):

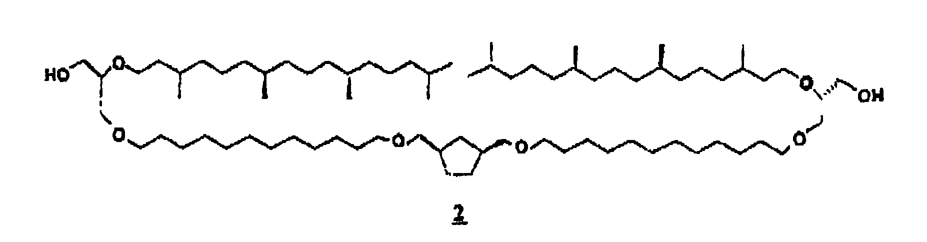

12. The compound for the synthesis of the compound according to formula (1) **characterised in that** it corresponds to formula (3):

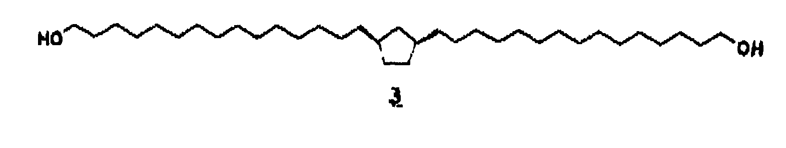

13. The compound for the synthesis of the compound according to formula (2) **characterised in that** it corresponds to formula (4):

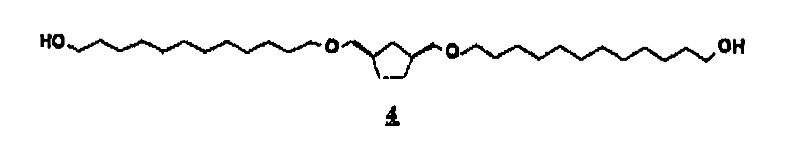

14. A liposomal composition incorporating at least one compound with the general formula (I) according to one of claims 1 to 11.

15. The composition according to claim 14
**characterised in that** it includes at least one colipid.

16. The composition according to claim 15
**characterised in that** said colipid is phosphatidylcholine from egg lecithin.

17. The composition according to claim 15 or 16 **characterised in that** it comprises a compound with the general formula I in which R₁ and R₂ are identical or different and each or both represent one of the following motifs:
- A₁-CH₂-N⁺(CH₃)₃, X⁻, X representing an halogen, A₁ representing an amide (NHC(O)) or ester (OC(O)) bond;
- A₂-(PEG_{X1}-A₃)ₙ-R₄, n being equal to 0 or 1, PEGₓ₁ being a polyethyleneglycol of molecular weight X₁, X₁ being less than or equal to 5,000 daltons, A₂ and A₃ being identical or different and representing one ether (O), ester (OC(O)), amide (NHC(O)), urea (NHC(O)NH), thiourea (NHC(S)NH), or thioether (S) bond, R₄ representing a targeting agent,
and at least one cationic colipid
and at least one fusogenic colipid.

18. The composition according to claim 17
**characterised in that** said cationic colipid is an MM12 or MM16 two-chain cationic lipid.

19. The composition according to claim 17 or 18 **characterised in that** said fusogenic colipid is dioleoylphosphatidylethanolamine or cholesterol.

20. The use of a liposomal composition according to any one of claims 14 to 19 for the delivery of molecules of therapeutic interest.

21. The use of a liposomal composition according to any one of claims 14 to 19 for the delivery of DNA or RNA molecules.

## Patentansprüche

1. Verbindung mit der allgemeinen Formel (I): 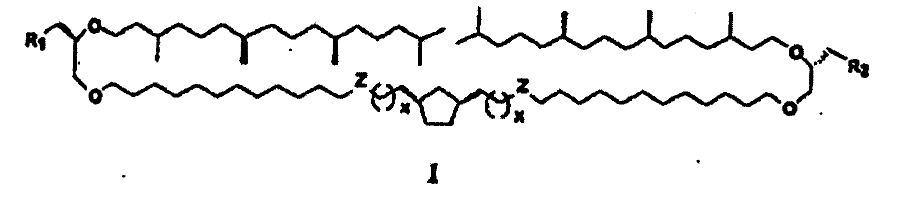 in der: .x gleich Null oder Eins ist,
. Z ein O, ein S oder ein CH₂ darstellt,
. R₁ und R₂, die identisch oder verschieden sein können, den einen oder den anderen der folgenden Substituenten darstellen:
. OH
. OY mit Y, das eine Schutzgruppe darstellt,
. OR₃, wobei R₃ einen Monosaccharid- oder Disaccharid-Substituenten darstellt,
. A₁-CH₂-N⁺(CH₃)₃, X⁻, wobei X ein Halogen darstellt und A₁ eine Amid-(HNC(O))- oder Ester-(OC(O))-verbindung darstellt,
. OPO(OM)₂, wobei M ein Alkalimetallkation- oder Erdalkalimetall darstellt,
. OP(O)O⁻-O(CH₂)₂-N⁺(CH₃)₃,
. A₂-(PEGₓ₁-A₃)ₙ-R4, wobei n gleich 0 oder 1 ist und PEGₓ₁ ein Polyethylenglykol mit einem Molekulargewicht X₁ ist, wobei X₁ kleiner oder gleich 5000 Daltons ist und A₂ und A₃ identisch oder verschieden sein können und eine Ether-(O)-, Ester-(OC(O))-, Amid-(NHC(O)), Harnstoff-(NHC(O)NH)-, Thioharnstoff-(NHC(S)NH)- oder Thioether-(S)-Verbindung darstellen können, wobei R₄ ein Zielagens darstellt.

2. Verbindung nach Anspruch 1, **dadurch gekennzeichnet, dass** die besagte Schutzgruppe eine Allyl- oder Benzyl- oder Tetrahydropyranyl- oder Trialkylsilylgruppe ist.

3. Verbindung nach Anspruch 1 oder 2, **dadurch gekennzeichnet, dass** das besagte Zielagens aus der Gruppe bestehend aus Folsäure, Mehrantennenstrukturen mit mehreren R₃-Einheiten, Antikörpern, Peptiden ausgewählt wird.

4. Verbindung nach einem der Ansprüche 1 bis 3, **dadurch gekennzeichnet, dass** R₃ ein Substituent ist, der aus der Gruppe bestehend aus den Substituenten D-Galaktosyl, D-Glukosyl, D-Mannosyl, Laktosyl, Maltosyl ausgewählt wird.

5. Verbindung nach einem der Ansprüche 1 oder 4, die der Formel (I) entspricht, in der:
. X gleich Null oder Eins ist,
. Z ein O oder ein CH₂ darstellt,
. R₁ und R₂ identisch oder verschieden sind und den einen oder den anderen der folgenden Substituenten darstellen:
. OH
. OR₃, wobei R₃ ein Laktosyl darstellt,
. OP(O)O⁻-O(CH₂)₂-N⁺(CH₃)₃,
. OPO(OM)₂, wobei M ein Alkalimetallkation- oder Erdalkalimetal darstellt,
. A₁-CH₂-N⁺(CH₃)₃, X⁻, wobei X ein Halogen darstellt und A₁ eine Amid-(HNC(O))- oder Ester-(OC(O))-verbindung darstellt.

6. Verbindung nach Anspruch 5, **dadurch gekennzeichnet, dass** sie der Formel (I) entspricht, in der R₁=R₂=OR₃, R₃=β-Laktosyl ist.

7. Verbindung nach Anspruch 1, **dadurch gekennzeichnet, dass** sie der Formel (I) entspricht, in der R₁=R₂=OP(O)O⁻-O(CH₂)₂-N⁺(CH₃)₃ ist.

8. Verbindung nach Anspruch 1, **dadurch gekennzeichnet, dass** sie der Formel (I) entspricht, in der R₁=R₂=NHC(O)CH₂-N⁺(CH₃)₃, X⁻ ist, wobei X ein Halogen darstellt.

9. Verbindung nach Anspruch 8, **dadurch gekennzeichnet, dass** X Chlor und Brom ist.

10. Verbindung nach Anspruch 1, **dadurch gekennzeichnet, dass** sie der Formel (1) entspricht:

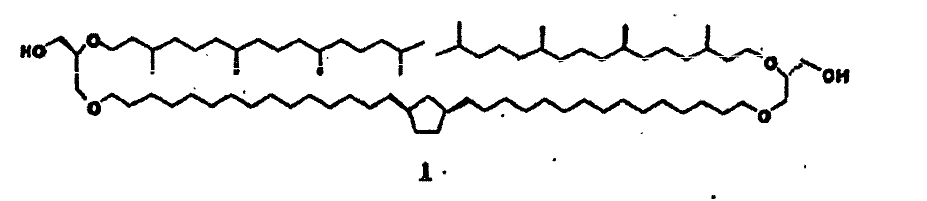

11. Verbindung nach Anspruch 1, **dadurch gekennzeichnet, dass** sie der Formel (2) entspricht:

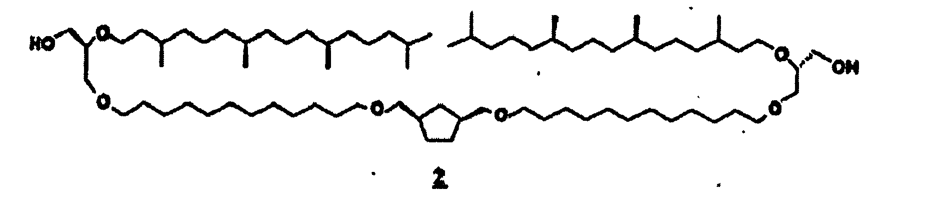

12. Verbindung für die Synthese der Verbindung nach Formel (1), **dadurch gekennzeichnet, dass** sie der Formel (3) entspricht:

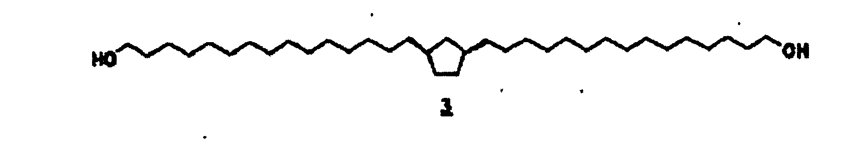

13. Verbindung für die Synthese der Verbindung nach Formel (2), **dadurch gekennzeichnet, dass** sie der Formel (4) entspricht:

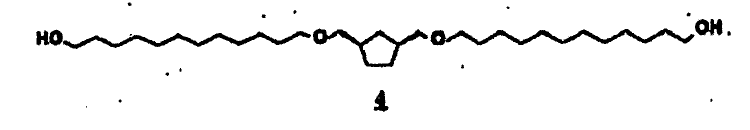

14. Liposom-Zusammensetzung, die mindestens eine Verbindung mit der allgemeinen Formel (I) nach einem der Ansprüche 1 bis 11 enthält.

15. Zusammensetzung nach Anspruch 14, **dadurch gekennzeichnet, dass** sie mindestens ein Kolipid aufweist.

16. Zusammensetzung nach Anspruch 15, **dadurch gekennzeichnet, dass** das besagte Kolipid das Phosphatidylcholin des Ei-Lecithins ist.

17. Zusammensetzung nach Anspruch 15 oder 16, **dadurch gekennzeichnet, dass** sie eine Verbindung mit der allgemeinen Formel **I** umfasst, in der R₁ und R₂ identisch oder verschieden sind und die eine und/oder die andere der folgenden Einheiten darstellen:
. A₁-CH₂-N⁺(CH₃)₃, X⁻, wobei X ein Halogen darstellt und A₁ eine Amid-(HNC(O))- oder Ester-(OC(O))-verbindung darstellt,
. A₂-(PEG_{X1}-A₃)ₙ-R₄, wobei n gleich 0 oder 1 ist und PEG_{X1} PEG_{X1} ein Polyethylenglykol mit dem Molekulargewicht X₁ ist, wobei X₁ kleiner oder gleich 5000 Daltons ist und A₂ und A₃ identisch oder verschieden sein können und eine Ether-(O)-, Ester-(OC(O))-, Amid-(NHC(O)), Harnstoff-(NHC(O)NH)-, Thioharnstoff-(NHC(S)NH)- oder Thioether-(S)-verbindung darstellen, wobei R₄ ein Zielagens darstellt,
und mindestens ein kationisches Kolipid
und mindestens ein fusogenisches Kolipid.

18. Verbindung nach Anspruch 17, **dadurch gekennzeichnet, dass** das besagte kationische Kolipid ein kationisches doppelsträngiges Lipid MM12 oder MM16 ist.

19. Verbindung nach Anspruch 17 oder Anspruch 18, **dadurch gekennzeichnet, dass** das besagte fusogenisches Kolipid Dioleylphosphatidylethanolamin oder Cholesterin ist.

20. Verwendung einer Liposom-Zusammensetzung nach einem der Ansprüche 14 bis 19 für die Vektorisierung von Molekülen von therapeutischem Interesse.

21. Verwendung einer Liposom-Zusammensetzung nach einem der Ansprüche 14 bis 19 für die Vektorisierung von ADN- oder ARN-Molekülen.
